# EUROPÄISCHE PATENTSCHRIFT

(11) **EP 3 769 833 B1**
(45) Veröffentlichungstag und Bekanntmachung des Hinweises auf die Patenterteilung: **20.04.2022**
(21) Anmeldenummer: 19188126.7
(22) Anmeldetag: 24.07.2019
(51) Int. Cl.: B01D 46/00, B01D 46/10, B01D 46/42, B01D 46/44, B01D 46/52

(54) **FILTERELEMENT MIT FUNKTIONSRAUM UND FILTERANORDNUNG MIT SOLCHEM FILTERELEMENT UND VERFAHREN ZUR STEUERUNG EINES FILTERSYSTEMS**
FILTER ELEMENT WITH FUNCTIONAL CHAMBER AND FILTER ARRANGEMENT WITH SUCH A FILTER ELEMENT AND METHOD FOR CONTROLLING A FILTER SYSTEM
ÉLÉMENT FILTRANT POURVU D'ESPACE FONCTIONNEL ET DISPOSITIF FILTRANT DOTÉ D'UN TEL ÉLÉMENT FILTRANT ET PROCÉDÉ DE COMMANDE D'UN SYSTÈME DE FILTRAGE

(43) Veröffentlichungstag der Anmeldung: 27.01.2021
(73) Patentinhaber: Carl Freudenberg KG, 69469 Weinheim (DE)
(72) Erfinder: OELSNER, Alexander, 68309 Mannheim (DE); BRÄUNLING, Volker, 64646 Heppenheim (DE); STAHL, Ulrich, 69514 Laudenbach (DE)

(56) Entgegenhaltungen:
- WO-A1-2007/093272
- DE-A1-102012 019 332
- KR-A- 20070 068 955

## Beschreibung

Die Erfindung betrifft ein Filterelement mit einem Funktionsraum gemäß dem Oberbegriff von Anspruch 1.

### Stand der Technik

Aus der WO 2007/093272 A1 ist ein Filterelement mit einem integrierten Behältnis bekannt. Das Behältnis kann dabei zur Aufnahme eines Beduftungselements dienen. Mit solchen Beduftungselementen kann ein Luftstrom im Filterelement mit einem bestimmten Duft versetzt werden.

Auch aus der US 2007/0261376 A1, der DE 10 2012 019 332 A1, der WO 2007/093272 A1 und der EP 2 868 987 A1 gehen Filterelemente mit Beduftungselementen hervor.

Aus der DE 10 2009 040 707 A1, der WO 2016/016085 A1, der KR 2007 0068955 A und der US 2003/0052791 A1 sind Filterelemente bekannt, die über Sensoren verfügen. Die Sensoren sind dabei direkt auf dem Filtermedium oder am Rahmen des Filters angebracht.

### Aufgabenstellung

Aufgabe der vorliegenden Erfindung ist es ein Filterelement zu schaffen, welches einen Funktionsraum aufweist, welcher in einfacher Art und Weise das Vorsehen von mindestens einem Sensorelement und/ oder von mindestens einem Beduftungselement und einen einfachen Austausch der Beduftungselemente ermöglicht.

Weitere Aufgabe ist es, eine Filteranordnung zu schaffen, welche einen einfachen Austausch eines Filterelements ermöglicht, insbesondere wenn das Filterelement ein wiederverwendbares Sensorelement umfasst.

### Technische Lösung

Gelöst wird diese Aufgabe durch ein Filterelement mit einem Funktionsraum mit den Merkmalen von Anspruch 1.

Erfindungsgemäß wurde als vorteilhaft erkannt den Funktionsraum innerhalb der Kantenstreifen des Filterelements vorzusehen, sodass das Filterelement bei zusätzlicher Funktionalität kein größeres Volumen aufweist und in bestehenden Filteranordnungen Verwendung finden kann.

Das erfindungsgemäße Filterelement besitzt einen Faltenbalg aus einem plissierten Filtermedium mit einer Vielzahl von Faltkanten und Faltflächen und besitzt Kantenstreifen zur Begrenzung des Filtermediums, sodass jeweils ein Kantenstreifen eine Wand des Filterelements bildet. Das Filterelement besitzt einen Funktionsraum, welcher fest mit dem Filterelement verbunden ist, an den Faltenbalg angrenzt und innerhalb der Kantenstreifen angeordnet ist. Eine Grenzfläche ist zwischen Faltenbalg und Funktionsraum angeordnet und trennt den Faltenbalg vom Funktionsraum. In vorteilhafter Weise ist die Grenzfläche mit mindestens einer Ausnehmung versehen, z.B. einer Aussparung oder einem Loch zur Aufnahme von Sensorelementen und/oder Beduftungselementen. Die Sensorelemente können dabei zur Messung von Partikeln, Gasen, Duftstoffen, etc. in einem Luftstrom durch das Filterelement dienen. Erfindungsgemäß sind die Sensorelemente und/oder Beduftungselemente in dem Filterelement nicht nur aufnehmbar sondern tatsächlich vorhanden und Teil des Filterelements.

Die Ausnehmungen sind dabei derart ausgestaltet, dass ein Sensorkopf des Sensorelements oder das Beduftungselement im Bereich des Faltenbalgs positionierbar ist. Damit ausreichend Platz für die Sensorelemente und/oder Beduftungselemente besteht, kann der Faltenabstand und/oder die Faltenhöhe des Faltenbalgs eventuell lokal so verändert werden, dass mehr Platz für Sensorelemente bzw. Beduftungselemente geschaffen wird. Eventuell vorhandene Beduftungselemente sind bevorzugt reingasseitig des Faltenbalgs angeordnet, da die Duftstoffe so direkt in die gereinigte Luft abgegeben werden können.

Die Grenzfläche des Filterelements ist dabei entweder aus dem Filtermedium ausgebildet oder als Kantenstreifen ausgeführt.

Das erfindungsgemäße Filterelement ist in vorteilhafter Weise besonders kompakt ausgestaltet und es wird eine einfache Bestückung mit Beduftungselementen bzw. Sensorelementen ermöglicht. Auch wird durch die Integration des Funktionsraumes in das Filterelement sichergestellt, dass die Anzahl möglicher Bypässe und damit die Gefahr von Fehlluft reduziert wird.

In vorteilhafter Weiterbildung des erfindungsgemäßen Filterelements sind mindestens zwei Ausnehmungen in der Grenzfläche vorgesehen zur Aufnahme mindestens eines Sensorelements in einer Ausnehmung und mindestens eines Beduftungselements in einer anderen Ausnehmung. Bei dieser Ausgestaltung kann in vorteilhafter Weise sowohl eine Beduftung des Luftstroms durch das Filterelement erfolgen als auch eine Messung bestimmter Eigenschaften des Luftstroms. Alle dazu erforderlichen Elemente sind in das Filterelement integriert.

In möglicher Ausgestaltung des erfindungsgemäßen Filterelements sind mindestens zwei Ausnehmungen in der Grenzfläche vorgesehen, zur Aufnahme von zwei Sensorelementen, wobei je eine Ausnehmung und damit auch je ein Sensorelement auf der Reingasseite und eine Ausnehmung und damit auch ein Sensorelement auf der Rohgasseite des Faltenbalgs positioniert ist. Dank einer derartigen Messung von Zuluft und Abluft sind Aussagen zur Luftqualität, Reinigungsleistung bzw. Filtrationsleistung, Lebensdauer des Filters, erforderlichem Filteraustausch, etc. möglich.

In einer alternativen Ausgestaltung oder auch Weiterbildung des erfindungsgemäßen Filterelements ist mindestens eine Ausnehmung 5b in der Grenzfläche 4 vorgesehen zur Aufnahme von mindestens einem Beduftungselement 7, wobei die mindestens eine Ausnehmung 5b auf der Reingasseite b des Faltenbalgs 1 positioniert ist, sodass ein direktes Beduften des gereinigten Luftstroms mit Duftstoff ermöglicht wird.

In einer alternativen Ausgestaltung oder auch Weiterbildung des erfindungsgemäßen Filterelements ist mindestens eine Ausnehmung 5b in der Grenzfläche 4 vorgesehen zur Aufnahme von mindestens einem Beduftungselement 7, wobei die mindestens eine Ausnehmung 5b auf der Rohgasseite a des Faltenbalgs 1 positioniert ist und das Beduftungselement einen Dosierstoff mit oxidierender Wirkung besitzt. Durch das rohgasseitige Beduftungselement kann ein Dosierstoff mit reinigender Wirkung für das Filtermedium des Faltenbalgs bereitgestellt werden.

In einer alternativen Ausgestaltung oder auch Weiterbildung des erfindungsgemäßen Filterelements ist mindestens eine Ausnehmung 5b in der Grenzfläche 4 vorgesehen zur Aufnahme von mindestens einem Beduftungselement 7, wobei die mindestens eine Ausnehmung 5b auf der Reingasseite b des Faltenbalgs 1 positioniert ist und das Beduftungselement einen Dosierstoff mit oxidierender Wirkung besitzt. Durch das reingasseitige Beduftungselement kann ein Dosierstoff mit reinigender Wirkung für dem Filterelement nachgeschaltete Komponenten, wie. z.B. Luftleitungen, Wärmetauscher, Steuerungsklappen, usw. bereitgestellt werden.

Bei Kombination dieser Ausgestaltungen ergibt sich ein Filterelement, bei welchem mindestens zwei Ausnehmungen in der Grenzfläche vorgesehen sind zur Aufnahme von zwei Beduftungselementen, wobei je ein Beduftungselement auf der Reingasseite und ein Beduftungselement auf der Rohgasseite des Faltenbalgs positioniert ist. Durch das reingasseitige Beduftungselement wird ein direktes Beduften des gereinigten Luftstroms mit Duftstoff ermöglicht. Durch das rohgasseitige Beduftungselement kann ein Dosierstoff für das Filtermedium des Faltenbalgs bereitgestellt werden. Durch ein weiteres reingasseitige Beduftungselement kann ein Dosierstoff mit reinigender Wirkung für dem Filterelement nachgeschaltete Komponenten bereitgestellt werden

Überraschenderweise wurde gefunden, dass ein Dosierstoff mit oxidierender Wirkung, wie z.B. eine mit Silikaten angereicherte Natriumhypochloritlösung eine reinigende Wirkung auf das Filtermedium bzw auf nachgeschaltete Komponenten in Bezug auf Mikroorganismen und Viren haben kann, was zu einer Verlängerung der Filterlebensdauer des Filterelements bzw. zu einer Verhinderung einer "Verseuchung" der nachgeschaltete Komponenten führt.

In vorteilhafter Ausgestaltung des erfindungsgemäßen Filterelements ist der Funktionsraum nur zu einer Seite hin geöffnet und ist damit nicht komplett abgeschlossen, sondern zugänglich. Insbesondere ist der Funktionsraum zur Rohgasseite hin geöffnet.

In einer ersten möglichen Ausführungsvariante des Filterelements erstreckt sich der Funktionsraum parallel zu den Faltkanten des Faltenbalgs und die Faltflächen des Faltenbalgs werden von eventuell vorhandenen Sensorelementen und/oder Beduftungselementen durchstoßen. Dazu sind auch in den Faltflächen des Faltenbalgs entsprechende Ausnehmungen vorhanden.

Gemäß der zweiten Ausführungsvariante ist der Funktionsraum angrenzend an die Stirnflächen der Falkanten des Faltenbalgs positioniert und die Grenzfläche ist als Kantenstreifen ausgeführt. Bei dieser Ausführungsvariante können eventuell vorhandene Sensorelemente und/oder Beduftungselemente zwischen bzw. oberhalb von Faltflächen liegen, sodass die Faltflächen des Faltenbalgs ganz bleiben.

In vorteilhafter Weiterbildung kann in dem Funktionsraum eine Sensorkomponente angeordnet sein, wobei die Sensorkomponente und das mindestens eine Sensorelement jeweils einen bzw. mehrere Sensoren ausbilden. So kann das Sensorelement im Wesentlichen den Messkopf umfassen und die Sensorkomponente die Ansteuerung und Stromversorgung des Sensorkopfes. Bei Sensorelement und Sensorkomponente kann es sich entweder um getrennte Elemente handeln. Sensorelement und Sensorkomponente können jedoch auch zu einem Bauteil zusammengefasst sein.

In vorteilhafter Weiterbildung ist in dem Funktionsraum zusätzlich ein Sender angeordnet, der datenübertragungstechnisch mit dem Sensor verbunden ist zur Datenübertragung von Messdaten des Sensors. Die Messdaten des Sensors können so an einen Empfänger übertragen werden, welcher beispielsweise in einem Gehäuse des Filterelements angebracht ist, oder in der Anlage bzw. Maschine, in welcher das Filterelement zum Einsatz kommt. Insbesondere kann der Sender als NFC Sender, als Nearfield-Communication-Sender ausgebildet sein, z.B. unter Verwendung von RFID oder "Bluetooth".

Die Erfindung betrifft auch eine Filteranordnung mit einem Rahmen und einem wie vorstehend beschriebenem Filterelement, wobei das Filterelement in den Rahmen aufgenommen ist. Unter Rahmen wird hierbei auch ein Gehäuse verstanden. Bei einer derartigen Ausgestaltung einer Filteranordnung wird in vorteilhafter Weise ein Funktionsraum innerhalb des Filterelements geschaffen. Der Rahmen muss nicht geändert werden und es besteht kein größerer Platzbedarf für das Filterelement. Auch wird keine zusätzliche Abdichtung des Funktionsraums zur Leckagevermeidung benötigt.

Weiterhin betrifft die Erfindung auch die Verwendung einer solchen Filteranordnung zur Luftfiltration in einer mobilen Einrichtung, insbesondere in einem Kraftfahrzeug oder einem Nutzfahrzeug.

Gegenstand der Erfindung ist auch ein Verfahren zur Steuerung eines Filtersystems mit einer wie obenstehend beschriebenen Filteranordnung. Das Filtersystem umfasst weiter eine Steuereinrichtung zur Ansteuerung des Filterelements, wobei die Steuereinrichtung datenübertragungstechnisch mit mindestens einem Sensor in dem Filterelement verbunden ist und die Ansteuerung des Filterelements abhängig von Messwerten des Sensors erfolgt. Beispielsweise kann die Ansteuerung des Filterelements die Steuerung des Volumenstroms, eine Ionisierung der Zuluft und eine Regulierung des Umluft-Frischluftanteils umfassen.

Die beschriebene Erfindung und die beschriebenen vorteilhaften Weiterbildungen der Erfindung stellen auch in Kombination miteinander - soweit dies technisch sinnvoll ist - vorteilhafte Weiterbildungen der Erfindung dar.

Hinsichtlich weiterer Vorteile und in konstruktiver und funktioneller Hinsicht vorteilhafter Ausgestaltungen der Erfindung wird auf die Unteransprüche sowie die Beschreibung von Ausführungsbeispielen unter Bezugnahme auf die beiliegenden Figuren verwiesen.

### Ausführungsbeispiel

Die Erfindung soll an Hand beigefügter Figuren noch näher erläutert werden. Einander entsprechende Elemente und Bauteile sind in den Figuren mit gleichen Bezugszeichen versehen. Zugunsten einer besseren Übersichtlichkeit der Figuren wurde auf eine maßstabsgetreue Darstellung verzichtet.

Es zeigen in schematischer Darstellung
- Fig. 1: eine erste Ausführungsvariante eines erfindungsgemäßen Filterelements
- Fig. 2: eine zweite Ausführungsvariante eines erfindungsgemäßen Filterelements
- Fig. 3a: ein Schnitt durch das Filterelement
- Fig. 3b: eine Ansicht der Trennfläche
- Fig. 4: eine Draufsicht einer Filteranordnung

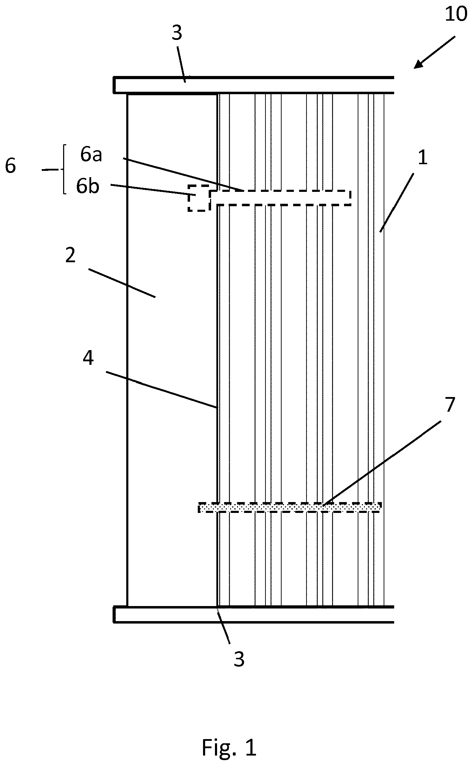

Fig. 1 zeigt ein Filterelement 10 in einer Draufsicht von der Rohgasseite her. Das Filterelement 10 besitzt einen Faltenbalg 1 aus plissiertem Filtermedium und direkt angrenzend an den Faltenbalg 1 einen Funktionsraum 2. Der Funktionsraum 2 erstreckt sich parallel zu den Faltkanten des Faltenbalgs 1. Der Faltenbalg 1 und der Funktionsraum 2 sind durch eine Grenzfläche 4 voneinander getrennt. Zur Stabilisierung und Abdichtung des Faltenbalgs 1 sind Kantenstreifen 3 vorgesehen. Der Funktionsraum 2 befindet sich innerhalb der Kantenstreifen 3 und ist fest mit dem Faltenbalg 1 verbunden und somit ein Teil des Filterelements 10. In der Grenzfläche 4 sind Ausnehmungen 5a, 5b vorgesehen, durch welche Sensorelemente 6a und Beduftungselemente 7 eingeführt und so eingeschoben werden können, dass sich diese im Bereich des Faltenbalgs 1 befinden. Eventuell vorhandene Sensorelemente 6a und Beduftungselemente 7 durchstoßen die Faltflächen den Faltenbalgs 1, wozu in den Faltflächen ebenfalls Ausnehmungen vorgesehen sind (nicht dargestellt). In der Darstellung von Fig. 1 ist ein solches Sensorelement 6a und ein solches Beduftungselement 7 mit gestrichelten Linien angedeutet. Einteilig mit dem Sensorelement 6a ist eine Sensorkomponente 6b verbunden, welche sich im Funktionsraum 2 befindet bzw. in diesen hineinragt. Das Sensorelement 6a und die Sensorkomponente 6b bilden zusammen einen Sensor 6 aus.

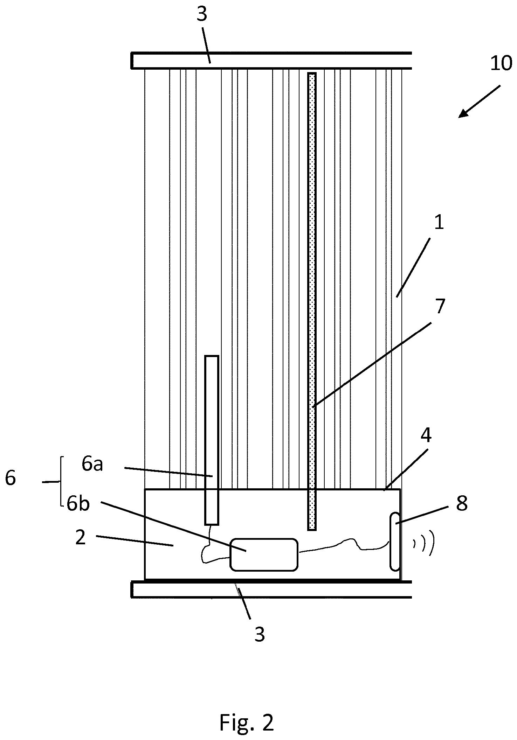

In Fig. 2 ist eine zweite alternative Anordnung des Funktionsraums 2 relativ zum Faltenbalg 1 dargestellt. Der Funktionsraum 2 grenzt an einer Seite an den Faltenbalg 1 an, nämlich dort, wo sich die Stirnflächen der Faltkanten des Faltenbalgs1 befinden. Durch eine Grenzfläche 4 wird der Funktionsraum 2 vom Faltenbalg 1 getrennt. Auch hier liegt der Funktionsraum 2 innerhalb der Grenzen der Kantenstreifen 3. Auch die Grenzfläche 4 kann als Kantenstreifen 3 ausgeführt sein. In der Draufsicht von Fig. 2 ist ein Sensor 6 und ein Beduftungselement 7 zu erkennen. Der Sensor 6 ist datenübertragungstechnisch mit einem Sender 8 verbunden, welcher Messdaten des Sensors 6 zu einem nicht dargestellten Empfänger übertragen kann.

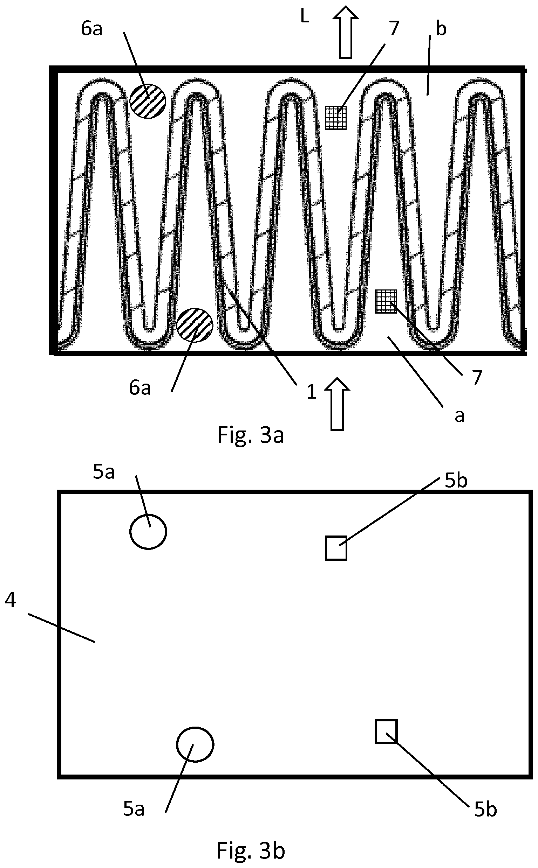

Während in den Fig. 1 und 2 jeweils nur ein Beduftungselement und ein - Sensorelement dargestellt waren, kann das Filterelement 10 auch mit mehreren Sensorelementen 6a und mehreren Beduftungselementen 7 bestückt sein. Dies ist in den Fig. 3a und 3b dargestellt.

Fig. 3a zeigt eine Schnittdarstellung durch das Filterelement 10 im Bereich seines Faltenbalgs 1. Der Faltenbalg 1 wird von einem Luftstrom L durchströmt. Auf der Rohgasseite a ist die Luft noch ungefiltert, auf der Reingasseite b ist die Luft bereits gefiltert. Das Filterelement 10 besitzt zwei Sensorelemente 6a und zwei Beduftungselemente 7. Ein erstes Sensorelement 6a ist auf der Rohgasseite a und ein zweites Sensorelement 6a auf der Reingasseite b angeordnet. Dadurch wird ermöglicht, die Luft vor und nach der Filterung durch das Filterelement 10 zu messen. Während das reingasseitige Beduftungselement 7 (bei b) einer direkten Beduftung des Luftstroms L dient, dienen die von dem rohgasseitigen (bei a) positionierten Beduftungselement 7 freigegebenen Dosierstoffe mit oxidierender Wirkung einer Reinigung des Filtermediums des Faltenbalgs 1, was zu einer Verlängerung der Filterlebensdauer führt.

In Fig. 3b ist die zugehörige Grenzfläche 4 dargestellt, welche über zwei Ausnehmungen 5a für die Sensorelemente 6a und über zwei Ausnehmungen 5b für die Beduftungselemente 7 verfügt. Die Ausnehmungen 5a bzw. 5b haben hier eine kreisförmige bzw. quadratische Grundform. Es sind jedoch auch andere Formen der Ausnehmungen 5a, 5b denkbar, beispielsweise auch nichtpunktsymmetrische Formen, welche ein Einschieben von Sensorelementen 6a und Beduftungselementen 7 nur in einer vorgegebenen Orientierung ermöglichen.

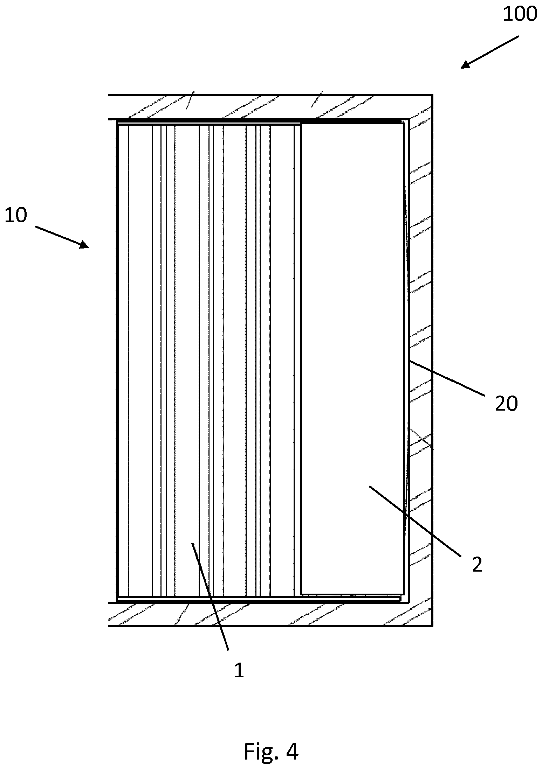

In Fig. 4 ist eine Filteranordnung 100 dargestellt, wobei ein Filterelement 10 von einem Rahmen 20 aufgenommen wird. Das Filterelement 10 besitzt - wie bereits obenstehend ausgeführt wird - einen Funktionsraum 2. Eventuelle vorhandene Sensorelemente 6a und Beduftungselemente 7 sind hier der besseren Übersichtlichkeit halber nicht dargestellt. Da der Funktionsraum 2 in das Filterelement 10 integriert ist und die Außenabmaße des Filterelements 10 trotz dem Vorhandensein des Funktionsraumes 2 unverändert bleiben können, kann das Filterelement 10 problemlos in Rahmen 20 in bestehende Maschinen und Anlagen montiert werden. Auch ergibt sich durch das Vorhandensein des Funktionsraums 2 kein zusätzliches Abdichtbedürfnis zwischen Filterelement 10 und Rahmen 20. Die zusätzlichen Funktionalitäten des Filterelements 10, welche durch die Sensorelemente 6a und die Beduftungselemente 7 geschaffen werden, können also ohne eine Veränderung des Systems um das Filterelement 10 herum, sprich ohne Veränderungen von Rahmen 20 und Filteranordnung 100 erfolgen.

### Bezugszeichenliste

- 1: Faltenbalg aus plissiertem Filtermedium
- 2: Funktionsraum
- 3: Kantenstreifen
- 4: Grenzfläche
- 5a: Ausnehmung Sensorelement
- 5b: Ausnehmung Beduftungselement
- 6: Sensor
- 6a: Sensorelement
- 6b: Sensorkomponente
- 7: Beduftungselement
- 8: Sender
- 9: -
- 10: Filterelement

- 20: Rahmen

- 100: Filteranordnung

- a: Rohgasseite
- b: Reingasseite
- L: Luftstrom

## Patentansprüche

1. Filterelement (10) mit einem Faltenbalg (1) aus einem plissierten Filtermedium und Kantenstreifen (3) zur Begrenzung des Faltenbalgs (1) wobei das Filterelement (10) einen Funktionsraum (2) besitzt, welcher an den Faltenbalg (1) angrenzt und innerhalb der Kantenstreifen (3) angeordnet ist, mit einer Grenzfläche (4) zwischen Faltenbalg (1) und Funktionsraum (2), welche den Faltenbalg (1) vom Funktionsraum (2) trennt,
wobei das Filterelement (10) mindestens ein Sensorelement (6a) und / oder mindestens ein Beduftungselement (7) besitzt,
**dadurch gekennzeichnet,**
**dass** die Grenzfläche (4) aus Filtermedium gebildet wird oder als Kantenstreifen (3) ausgeführt ist, und
**dass** die Grenzfläche (4) mit mindestens einer Ausnehmung (5a, 5b) versehen ist zur Aufnahme der Sensorelemente (6a) und/oder der Beduftungselemente (7).

2. Filterelement nach Anspruch 1 **dadurch gekennzeichnet**,
das Filterelement (10) mindestens ein Sensorelement (6a) und mindestens ein Beduftungselement (7) besitzt und,
dass mindestens zwei Ausnehmungen (5a, 5b) in der Grenzfläche (4) vorgesehen sind zur Aufnahme mindestens eines Sensorelements (6a) und mindestens eines Beduftungselements (7).

3. Filterelement nach einem der vorangehenden Ansprüche **dadurch gekennzeichnet**,
das Filterelement (10) mindestens zwei Sensorelemente (6a) besitzt und, dass mindestens zwei Ausnehmungen (5a) in der Grenzfläche (4) vorgesehen sind zur Aufnahme von mindestens zwei Sensorelementen (6a), wobei mindestens je eine Ausnehmung (5a) auf der Reingasseite (b) und eine Ausnehmung (5a) auf der Rohgasseite (a) des Faltenbalgs (1) positioniert ist.

4. Filterelement nach einem der vorangehenden Ansprüche **dadurch gekennzeichnet**,
das Filterelement (10) mindestens ein Beduftungselement (7) besitzt und,
- dass mindestens eine Ausnehmung (5b) in der Grenzfläche (4) vorgesehen ist zur Aufnahme von mindestens einem Beduftungselement (7), wobei die mindestens eine Ausnehmung (5b) auf der Reingasseite (b) des Faltenbalgs (1) positioniert ist, und/oder
- dass mindestens eine Ausnehmung (5b) in der Grenzfläche (4) vorgesehen ist zur Aufnahme von mindestens einem Beduftungselement (7), wobei die mindestens eine Ausnehmung (5b) auf der Rohgasseite (a) des Faltenbalgs (1) positioniert ist und das Beduftungselement einen Dosierstoff mit oxidierender Wirkung besitzt, und/oder
- dass mindestens eine Ausnehmung (5b) in der Grenzfläche (4) vorgesehen ist zur Aufnahme von mindestens einem Beduftungselement (7), wobei die mindestens eine Ausnehmung (5b) auf der Reingasseite (b) des Faltenbalgs (1) positioniert ist und das Beduftungselement einen Dosierstoff mit oxidierender Wirkung besitzt.

5. Filterelement nach einem der vorangehenden Ansprüche **dadurch gekennzeichnet,**
**dass** der Funktionsraum (2) nur zu einer Seite hin geöffnet ist, insbesondere zur Rohgasseite (a) hin.

6. Filterelement nach einem der vorangehenden Ansprüche **dadurch gekennzeichnet,**
**dass** sich der Funktionsraum (2) parallel zu den Faltkanten des Faltenbalgs (1) erstreckt und die Faltflächen von Sensorelementen (6a) und /oder Beduftungselementen (7) durchstoßen werden.

7. .Filterelement nach einem der Ansprüche 1 bis 5 **dadurch gekennzeichnet,**
**dass** der Funktionsraum (2) angrenzend an die Stirnflächen der Faltkanten des Faltenbalgs (1) positioniert ist und die Grenzfläche (4) als Kantenstreifen (3) ausgeführt ist.

8. Filterelement nach einem der vorangehenden Ansprüche **dadurch gekennzeichnet,**
**dass** das Filterelement (10) mindestens ein Sensorelement (6a) besitzt und,
**dass** in dem Funktionsraum (4) eine Sensorkomponente (6b) angeordnet ist, wobei die Sensorkomponente (6b) und das Sensorelement (6a) einen Sensor (6) bilden.

9. Filterelement nach Anspruch 8 **dadurch gekennzeichnet,**
**dass** in dem Funktionsraum (2) ein Sender (8) angeordnet ist, der datenübertragungstechnisch mit dem Sensor (6) verbunden ist zur Datenübertragung von Messdaten des Sensors (6).

10. Filteranordnung (100) mit einem Rahmen (20) und einem Filterelement (10) nach einem der vorangehenden Ansprüche, wobei das Filterelement (10) in dem Rahmen (20) aufgenommen ist.

11. Verwendung einer Filteranordnung (100) nach Anspruch 10 zur Luftfiltration in einer mobilen Einrichtung, insbesondere in einem Kraftfahrzeug oder einem Nutzfahrzeug.

12. Verfahren zur Steuerung eines Filtersystems mit einer Filteranordnung (100) nach Anspruch 10 und einer Steuereinrichtung zur Ansteuerung des Filterelements (10), wobei die Steuereinrichtung datenübertragungstechnisch mit mindestens einem Sensor (6) in dem Filterelement (10) verbunden ist und die Ansteuerung des Filterelements (10) abhängig von Messwerten des Sensors (6) erfolgt.

## Claims

1. Filter element (10) having a bellows (1) made of a pleated filter medium and edge strips (3) for bounding the bellows (1), wherein the filter element (10) has a functional space (2) that adjoins the bellows (1) and is arranged within the edge strips (3), having an interface (4) between the bellows (1) and functional space (2), said interface (4) separating the bellows (1) from the functional space (2),
wherein the filter element (10) has at least one sensor element (6a) and/or at least one fragrancing element (7),
**characterized**
**in that** the interface (4) is formed from filter medium or is embodied as an edge strip (3), and
**in that** the interface (4) is provided with at least one cutout (5a, 5b) for receiving the sensor elements (6a) and/or the fragrancing elements (7).

2. Filter element according to Claim 1, characterized the filter element (10) has at least one sensor element (6a) and at least one fragrancing element (7), and
in that at least two cutouts (5a, 5b) for receiving at least one sensor element (6a) and at least one fragrancing element (7) are provided in the interface (4).

3. Filter element according to either of the preceding claims, characterized
the filter element (10) has at least two sensor elements (6a), and
in that at least two cutouts (5a) for receiving at least two sensor elements (6a) are provided in the interface (4), wherein at least one cutout (5a) is positioned on the clean gas side (b) and at least one cutout (5a) is provided on the raw gas side (a) of the bellows (1).

4. Filter element according to one of the preceding claims, characterized
the filter element (10) has at least one fragrancing element (7), and
- in that at least one cutout (5b) for receiving at least one fragrancing element (7) is provided in the interface (4), wherein the at least one cutout (5b) is positioned on the clean gas side (b) of the bellows (1), and/or
- in that at least one cutout (5b) for receiving at least one fragrancing element (7) is provided in the interface (4), wherein the at least one cutout (5b) is positioned on the raw gas side (a) of the bellows (1) and the fragrancing element exhibits a metered substance having an oxidizing action, and/or
- in that at least one cutout (5b) for receiving at least one fragrancing element (7) is provided in the interface (4), wherein the at least one cutout (5b) is positioned on the clean gas side (b) of the bellows (1) and the fragrancing element exhibits a metered substance having an oxidizing action.

5. Filter element according to one of the preceding claims, **characterized**
**in that** the functional space (2) is open only towards one side, in particular towards the raw gas side (a).

6. Filter element according to one of the preceding claims, **characterized**
**in that** the functional space (2) extends parallel to the fold edges of the bellows (1) and the fold surfaces are penetrated by sensor elements (6a) and/or fragrancing elements (7).

7. Filter element according to one of Claims 1 to 5, **characterized**
**in that** the functional space (2) is positioned in a manner adjoining the end faces of the fold edges of the bellows (1) and the interface (4) is embodied as an edge strip (3).

8. Filter element according to one of the preceding claims, **characterized**
**in that** the filter element (10) has at least one sensor element (6a), and
**in that** a sensor component (6b) is arranged in the functional space (4), wherein the sensor component (6b) and the sensor element (6a) form a sensor (6).

9. Filter element according to Claim 8, **characterized in that** a transmitter (8) is arranged in the functional space (2), said transmitter (8) being connected to the sensor (6) in terms of data transmission for the data transmission of measurement data from the sensor (6).

10. Filter arrangement (100) having a frame (20) and a filter element (10) according to one of the preceding claims, wherein the filter element (10) is accommodated in the frame (20).

11. Use of a filter arrangement (100) according to Claim 10 for air filtration in a mobile piece of equipment, in particular in a motor vehicle or commercial vehicle.

12. Method for controlling a filter system having a filter arrangement (100) according to Claim 10 and a control device for controlling the filter element (10), wherein the control device is connected to at least one sensor (6) in the filter element (10) in terms of data transmission and the filter element (10) is controlled on the basis of measured values from the sensor (6).

## Revendications

1. Élément filtrant (10) comprenant un soufflet (1) constitué d'un milieu filtrant plissé et de bandes de bordure (3) destinées à délimiter le soufflet (1), l'élément filtrant (10) possédant un espace fonctionnel (2) qui est adjacent au soufflet (1) et qui est disposée à l'intérieur des bandes de bordure (3), une interface (4) située entre le soufflet (1) et l'espace fonctionnel (2) et séparant le soufflet (1) de l'espace fonctionnel (2),
l'élément filtrant (10) possédant au moins un élément de capteur (6a) et/ou au moins un élément odorant (7), **caractérisé en ce que**
l'interface (4) est formée d'un milieu filtrant ou est réalisée sous la forme d'une bande de bordure (3), et l'interface (4) est pourvue d'au moins un évidement (5a, 5b) destiné à recevoir les éléments formant capteurs (6a) et/ou les éléments odorants (7).

2. Élément filtrant selon la revendication 1, caractérisé
l'élément filtrant (10) possède au moins un élément de capteur (6a) et au moins un élément odorant (7) et
au moins deux évidements (5a, 5b) sont prévus dans l'interface (4) pour recevoir au moins un élément de capteur (6a) et au moins un élément odorant (7).

3. Élément filtrant selon l'une des revendications précédentes, caractérisé
l'élément filtrant (10) possède au moins deux éléments formant capteurs (6a) et
au moins deux évidements (5a) sont prévus dans l'interface (4) pour recevoir au moins deux éléments formant capteurs (6a), au moins un évidement (5a) étant positionné du côté gaz propre (b) et un évidement (5a) étant positionné du côté gaz brut (a) du soufflet (1).

4. Élément filtrant selon l'une des revendications précédentes, caractérisé
l'élément filtrant (10) possède au moins un élément odorant (7) et,
- au moins un évidement (5b) est prévu dans l'interface (4) pour recevoir au moins un élément odorant (7), l'au moins un évidement (5b) étant positionné du côté gaz propre (b) du soufflet (1), et/ou
- au moins un évidement (5b) est prévu dans l'interface (4) pour recevoir au moins un élément odorant (7), l'au moins un évidement (5b) étant positionné du côté gaz brut (a) du soufflet (1) et l'élément odorant possédant une substance de dosage à effet oxydant, et/ou
- au moins un évidement (5b) est prévu dans l'interface (4) pour recevoir au moins un élément odorant (7), ledit au moins un évidement (5b) étant positionné du côté gaz propre (b) du soufflet (1) et l'élément odorant possédant une substance de dosage à effet oxydant.

5. Élément filtrant selon l'une des revendications précédentes, **caractérisé en ce que**
l'espace fonctionnel (2) n'est ouvert que d'un côté, en particulier du côté gaz brut (a).

6. Élément filtrant selon l'une des revendications précédentes, **caractérisé en ce que**
l'espace fonctionnel (2) s'étend parallèlement aux bords de pliage du soufflet (1) et les surfaces de pliage sont percées par des éléments formant capteurs (6a) et/ou des éléments odorants (7).

7. Élément filtrant selon l'une des revendications 1 à 5, **caractérisé en ce que**
l'espace fonctionnel (2) est positionné de manière adjacentes aux surfaces frontales des bords de pliage du soufflet (1) et l'interface (4) est conçue comme une bande de bordure (3).

8. Élément filtrant selon l'une des revendications précédentes, **caractérisé en ce que**
l'élément filtrant (10) possède au moins un élément de capteur (6a) et,
un composant de capteur (6b) est disposé dans l'espace fonctionnel (4), le composant de capteur (6b) et l'élément de capteur (6a) formant un capteur (6).

9. Élément filtrant selon la revendication 8, **caractérisé en ce que**
un émetteur (8) est disposé dans l'espace fonctionnel (2) et est relié au capteur (6) par une technique de transmission de données afin de transmettre des données de mesure du capteur (6).

10. Ensemble de filtration (100) comprenant un cadre (20) et un élément filtrant (10) selon l'une des revendications précédentes, l'élément filtrant (10) étant reçu dans le cadre (20).

11. Utilisation d'un ensemble de filtration (100) selon la revendication 10 pour filtrer l'air dans un dispositif mobile, notamment dans un véhicule automobile ou un véhicule utilitaire.

12. Procédé de commande d'un système de filtration comprenant un ensemble de filtration (100) selon la revendication 10 et un dispositif de commande destiné à commander l'élément filtrant (10), le dispositif de commande étant relié, par une technique de transmission de données, à au moins un capteur (6) situé dans l'élément filtrant (10) et la commande de l'élément filtrant (10) étant effectuée en fonction de valeurs de mesure du capteur (6).
